Europäisches Patentamt

European Patent Office

Office européen des brevets

(11) Numéro de publication: **0 255 156 B1**

(12) **FASCICULE DE BREVET EUROPEEN**

(45) Date de publication de fascicule du brevet:
**16.10.91**

(51) Int. Cl.⁵: **C07C 19/045**, C07C 17/156,
B01J 27/138

(21) Numéro de dépôt: **87201160.6**

(22) Date de dépôt: **17.06.87**

(54) Procédé d'oxychloration de l'éthylène et compositions catalytiques pour l'oxychloration.

(30) Priorité: **27.06.86 FR 8609504**

(43) Date de publication de la demande:
**03.02.88 Bulletin 88/05**

(45) Mention de la délivrance du brevet:
**16.10.91 Bulletin 91/42**

(84) Etats contractants désignés:
**AT BE CH DE ES FR GB IT LI LU NL SE**

(56) Documents cités:
FR-A- 2 095 351
GB-A- 969 937
US-A- 3 468 968
US-A- 4 460 699

(73) Titulaire: **SOLVAY & Cie (Société Anonyme)**
**Rue du Prince Albert, 33**
**B-1050 Bruxelles(BE)**

(72) Inventeur: **Derleth, Helmut**
**Grosse Drakenburger Strasse 58**
**W-3070 Nienburg(DE)**
Inventeur: **Walter, Robert**
**Hindenburgstrasse 64**
**W-3012 Langenhagen(DE)**
Inventeur: **Weidenbach, Günter**
**Lehrter Strasse 52**
**W-3000 Hannover73(DE)**
Inventeur: **Strebbele, Michel**
**Rue Sombre 84**
**B-1150 Bruxelles(BE)**

(74) Mandataire: **Nichels, William et al**
**Solvay & Cie Département de la Propriété**
**Industrielle Rue de Ransbeek, 310**
**B-1120 Bruxelles(BE)**

Rank Xerox (UK) Business Services

## Description

La présente invention se rapporte à un procédé d'oxychloration de l'éthylène à l'intervention d'une composition catalytique permettant d'obtenir un rendement amélioré et engendrant simultanément un effet de corrosion amoindri des réacteurs en acier inoxydable notamment par une réduction du collage et mottage (agglomération) des grains de catalyseur.

La chloration d'hydrocarbures gazeux à l'intervention de chlorure d'hydrogène et d'air, ou d'oxygène, est une opération qui est connue de longue date. Cette chloration s'effectue habituellement en présence de catalyseurs constitués de sels métalliques déposés sur des supports inertes. Comme sels métalliques, on utilise généralement des halogénures tels que le chlorure cuivrique qui, lorsqu'il est utilisé seul, présente toutefois l'inconvénient d'être relativement volatil, ce qui entraîne une chute de l'activité catalytique et du rendement de la réaction d'oxychloration, inacceptable industriellement, lorsque ce composé est utilisé en lit fluidisé dans une installation industrielle.

Pour remédier à ces inconvénients, on a proposé d'incorporer dans les compositions catalytiques d'autres sels métalliques tels que les chlorures des métaux alcalins, alcalino-terreux et autres métaux dérivés des terres rares ou lanthanides (brevet britannique GB-969937, brevet belge BE-741055).

Ces différentes compositions catalytiques présentent des activités dépendant notamment de la nature de l'hydrocarbure que l'on veut mettre en oeuvre et de la nature du sel ou des sels métalliques ajoutés pour diminuer les effets de la volatilité du chlorure cuivrique. En outre, ces différentes compositions catalytiques engendrent également des effets de corrosion de l'acier des réacteurs industriels provoqués notamment par le collage sur les parois et le mottage des grains de catalyseur. Cette corrosion est un des effets qui, dans l'état actuel des connaissances, ne peut être déduit ni qualitativement, ni quantitativement, des données techniques en rapport avec la nature des sels mis en oeuvre et qui doit être étudié de façon empirique pour chaque composition catalytique envisagée.

On a maintenant trouvé des compositions catalytiques à base de cuivre et de magnésium permettant de fabriquer du 1,2-dichloréthane par oxychloration de l'éthylène avec une activité et un rendement qui n'ont jamais été atteints industriellement avec des compositions comprenant ces deux métaux. Ces compositions engendrent simultanément un collage et mottage minimal des grains de catalyseur ainsi qu'un minimum de corrosion du réacteur industriel.

Par conséquent, la présente invention concerne un procédé d'oxychloration de l'éthylène en 1,2-dichloréthane dans lequel la réaction d'oxychloration est catalysée par une composition catalytique comprenant un support d'alumine sur lequel est déposé du chlorure cuivrique, du chlorure de magnésium et un chlorure d'un métal alcalin choisi parmi le chlorure de sodium et le chlorure de lithium.

Lorsque le métal alcalin mis en oeuvre est le sodium, on opère avec des rapports atomiques en sels de cuivre et de sodium de 1 : 0,01-0,2. En effet, il a été constaté que dans le cas des sels de sodium, on observe une corrosion des réacteurs en acier inoxydable qui devient importante, voire inacceptable lorsque l'on dépasse des rapports atomiques Cu : Na de 1 : 0,2.

Par contre, si le métal alcalin est le lithium, le rapport atomique Cu : Li est moins critique et on opère avec des rapports Cu : Li allant de 1 : 0,0001 à 1 : 2.

L'invention concerne également des compositions catalytiques pour l'oxychloration d'hydrocarbures comprenant, soit

1) du chlorure cuivrique, du chlorure de magnésium et du NaCl dans des rapports atomiques compris entre

1 : 0,1-1,5 : 0,01-0,2 déposés sur de l'alumine, soit

2) du chlorure cuivrique, du chlorure de magnésium et du LiCl déposés sur alumine. Dans ce cas, les rapports entre les différents sels sont habituellement compris entre

1 : 0,1-1,5 : 0,0001-1,5 et de préférence entre

1 : 0,3-1 : 0,001-1.

L'alumine mise en oeuvre dans les compositions catalytiques de l'invention peut être de toute origine et être obtenue selon tout procédé connu; on utilise habituellement des alumines de type eta ou gamma. De bons résultats ont été obtenus avec une alumine de type gamma.

L'alumine généralement mise en oeuvre dans les compositions catalytiques de l'invention, présente un diamètre moyen des particules compris entre 20 et 100 $\mu$ et de préférence un diamètre moyen compris entre 25 et 75 $\mu$.

La surface spécifique de l'alumine mesurée suivant la méthode B.E.T. est généralement comprise entre 50 m$^2$/g et 250 m$^2$/g. De bons résultats ont été obtenus avec une alumine dont la surface spécifique était comprise entre 100 m$^2$/g et 210 m$^2$/g.

Enfin, le volume poreux des alumines habituellement utilisées se situe entre 0,1 et 1 cm$^3$/g. De

EP 0 255 156 B1

préférence, le volume poreux est compris entre 0,2 et 0,8 cm³/g et de bons résultats ont été obtenus avec une alumine dont le volume poreux se situe entre 0,3 et 0,6 cm³/g.

Il est à noter que l'alumine, de par sa nature, contient une quantité plus ou moins grande d'atomes de sodium pouvant être intégrés dans le réseau cristallin ou être liés sous une forme chimique quelconque. La présence de ces atomes de sodium, que l'on peut plus aisément qualifier "d'atomes de sodium non lavables", n'a aucune importance pour la présente invention qui se rapporte uniquement à des compositions catalytiques qui contiennent un métal alcalin, sous forme de sel, pouvant être considéré comme constituant un "métal alcalin lavable". Ces sels alcalins sont non liés chimiquement au support d'alumine et sont généralement introduits dans les compositions catalytiques par imprégnation de l'alumine avec le sel considéré. Cette imprégnation avec le sel alcalin désiré peut se faire, soit en même temps que l'imprégnation avec les autres sels, soit avant, soit après cette imprégnation.

Le mode d'obtention des compositions catalytiques selon l'invention n'est pas critique en soi pour autant que les compositions catalytiques finalement obtenues répondent aux caractéristiques exposées ci-avant. Un mode d'obtention ayant donné de bons résultats consiste à imprégner une alumine $\gamma$ ayant les caractéristiques suivantes :

- diamètre moyen des particules d'environ 50 $\mu$,
- surface spécifique B.E.T. comprise entre 170 et 190 m²/g,
- volume poreux environ 0,4 cm³/g,
- poids spécifique par écoulement libre de 0,75 kg/dm³.

Cette imprégnation se fait en une seule étape à une température de 75°C avec une solution aqueuse renfermant les quantités désirées de chlorures de cuivre, de magnésium et de métal alcalin. On évite l'apparition d'une phase liquide non adsorbée par le solide en limitant le volume de la solution imprégnante à 70 à 100 % du volume poreux de la quantité d'alumine mise en oeuvre. Cette alumine imprégnée est ensuite séchée avant de l'introduire dans le réacteur d'oxychloration proprement dit.

Les compositions catalytiques finalement mises en oeuvre dans le procédé d'oxychloration présentent généralement une teneur en cuivre, calculée sous forme métallique, comprise entre 30 g/kg et 90 g/kg. De préférence, cette quantité se situe entre 50 et 75 g/kg et tout particulièrement préférées sont des compositions catalytiques dont la teneur en cuivre est comprise entre 60 et 70 g/kg.

La teneur des autres sels dans les compositions catalytiques quant à elle peut aisément être déduite des teneurs en cuivre ainsi spécifiées en combinaison avec les rapports atomiques déjà précisés ci-avant.

Les compositions catalytiques finales présentent généralement une surface spécifique B.E.T. comprise entre 25 m²/g et 200 m²/g et de préférence entre 50 et 150 m²/g. De bons résultats ont été obtenus avec des compositions catalytiques dont la surface spécifique B.E.T. est comprise entre 100 et 140 m²/g.

Les compositions catalytiques peuvent être mises en oeuvre dans tout procédé d'oxychloration réalisé avec un catalyseur disposé en lit fixe ou en lit fluidisé. Elles sont particulièrement avantageuses dans un procédé dans lequel le catalyseur est sous forme de lit fluidisé du fait d'un rendement amélioré et de l'absence de corrosion dans les réacteurs en acier inoxydable.

Lorsque l'on opère avec un catalyseur disposé en lit fluidisé, la température à laquelle s'effectue la réaction d'oxychloration se situe habituellement entre 200 et 300°C. De préférence, cette température est comprise entre 220 et 280°C. Enfin, des bons résultats ont été obtenus avec une température située aux environs de 240°C-270°C.

La pression à laquelle est effectuée la réaction d'oxychloration n'est pas critique en elle-même. Habituellement, on opère avec des pressions comprises entre 1 et 10 atm et de préférence avec des pressions comprises entre 1 et 8 atm.

La vitesse de fluidisation des compositions catalytiques n'est par critique en elle-même et dépend essentiellement de la granulométrie du catalyseur et des dimensions de l'appareillage. Généralement, on opère avec des vitesses comprises entre 5 et 100 cm/s et de préférence entre 10 cm/s et 50 cm/s.

Enfin, le rapport des réactifs mis en oeuvre est le même que celui généralement utilisé dans les procédés antérieurs. D'habitude, on opère avec un léger excès d'éthylène par rapport à la quantité d'HCl mise en oeuvre. Toutefois, les compositions catalytiques de l'invention permettent également de travailler au voisinage de la stoechiométrie, voire même en excès d'HCl.

L'invention se trouve plus amplement illustrée par les exemples suivants.

Exemple 1

225 cm³ d'une composition catalytique, constituée d'une alumine de type gamma, présentant une surface spécifique moyenne de 190 m²/g, imprégnée avec une solution aqueuse de chlorures de cuivre, de magnésium et de sodium, sont disposés après séchage dans un réacteur en Inconel 600, d'oxychloration

3

de l'éthylène en lit fluide en 1,2-dichloréthane et équipé de plaquettes en alliage inoxydable austénitique de type AISI 316 L.

Dans ce réacteur, les gaz réactifs sont introduits par le bas à travers un filtre métallique fritté. Les produits de réaction sont ensuite détendus jusqu'à la pression atmosphérique par une vanne de régulation de pression du réacteur. Les produits de réaction sont ensuite refroidis dans un piège maintenu à - 20°C et les gaz non condensés sont lavés dans un scrubber à eau avant de balayer une ampoule de prélèvement. Le bilan des produits formés est effectué au départ d'analyses chromatographiques des produits liquide et gazeux recueillis et du titrage de l'acidité de la solution aqueuse recueillie au pied du scrubber.

4 essais sont réalisés avec des compositions catalytiques contenant des quantités différentes de sodium telles que reprises au Tableau 1 ci-après. Ce Tableau 1 comprend également les conditions de fonctionnement du réacteur et les résultats obtenus.

Les rendements obtenus dans ces essais de laboratoire sont limités par le temps de séjour restreint de 5 s. La conversion de $C_2H_4$ est, dès lors, limitée dans tous les essais (les conditions de marche d'un réacteur industriel permettent d'atteindre des temps de séjour nettement plus longs : 10 à 80 s, et le plus souvent de 20 à 50 s afin d'assurer une meilleure conversion de l'éthylène).

Tableau 1

| ESSAI | 1 | 2 | 3 | 4 |
|---|---|---|---|---|
| **I. Catalyseur** | | | | |
| **a) support :** | | | | |
| alumine : type | $\gamma$ | $\gamma$ | $\gamma$ | $\gamma$ |
| : surf. spécifique ($m^2/g$) | 190 | 190 | 186 | 190 |
| **b) composition catalytique** | | | | |
| Cu en g/kg | 57 | 60 | 58 | 58,5 |
| Mg en g/kg | 16,5 | 17 | 17,7 | 16,2 |
| Na en g/kg | 0,1 | 1,0 | 1,7 | 6,2 |
| Surface spécifique BET $m^2/g$ | 120 | 122 | 124 | 113 |
| **II. Conditions opératoires de l'oxychloration** | | | | |
| HCl : lN/h | 160 | 160 | 160 | 160 |
| air : lN/h | 260 | 260 | 260 | 260 |
| $C_2H_4$ : lN/h | 84 | 84 | 84 | 84 |
| t° : °C * | 255 | 255 | 255 | 255 |
| pression : bars absolus | 6 | 6 | 6 | 6 |
| vitesse de fluidisation (cm/s) | 10 | 10 | 10 | 10 |
| temps de contact : s | 5 | 5 | 5 | 5 |

* Profil de température stable dans le réacteur : la température du lit fluide ne s'écarte pas de plus de 2°C de la température moyenne.

EP 0 255 156 B1

**Tableau 1** (suite)

| ESSAI | 1 | 2 | 3 | 4 |
|---|---|---|---|---|
| RENDEMENT : | | | | |
| 1,2-DCEa (% mol) $C_2H_4$ mis en oeuvre | 90,5 | 91,5 | 91,8 | 91,8 |
| Conversion de $C_2H_4$ % mol. | 95,5 | 96 | 96 | 95 |
| Formation de : $C_2H_5Cl$ % mol. par rapport au $C_2H_4$ mis en oeuvre | 1,6 | 0,6 | 0,5 | 0,6 |
| Corrosion de l'acier AISI 316 L | faible (fig. 1) | faible (fig. 2) | faible (fig. 3) | forte (fig. 4) |

On peut déduire de la comparaison des résultats des différents essais qui :
- lorsque l'on opère en dessous des limites en sodium de la présente invention (essai 1), le rendement en 1,2-dichloréthane est inférieur à celui obtenu avec les compositions catalytiques de l'invention (essais 2 et 3). Par contre, la corrosion de l'acier est faible et acceptable;
- lorsque l'on opère au-dessus des limites en sodium de la présente invention (essai 4), le rendement en 1,2-dichloréthane est excellent et comparable à celui obtenu selon l'invention (essais 2 et 3). Par contre, la corrosion de l'acier est forte et donc inacceptable d'un point de vue industriel;
- lorsque l'on opère dans les limites en sodium de la présente invention (essais 2 et 3), on observe simultanément un excellent rendement en 1,2-dichloréthane et une corrosion faible et acceptable de l'acier.

Exemple 2

225 cm³ d'une composition catalytique, constituée d'une alumine du type gamma, présentant une surface spécifique moyenne de 180 à 190 m²/g, imprégnée avec une solution aqueuse de chlorures de cuivre, de magnésium et de lithium, sont disposés après séchage dans un réacteur en Inconel 600, d'oxychloration de l'éthylène en lit fluide en 1,2-dichloréthane et équipé de plaquettes en alliage inoxydable austénitique de type AISI 316 L.

Dans ce réacteur, les gaz réactifs sont introduits par le bas à travers un filtre métallique fritté. Les produits de réaction sont ensuite détendus jusqu'à la pression atmosphérique par une vanne de régulation de pression du réacteur. Les produits de réaction sont ensuite refroidis dans un piège maintenu à - 20° C et les gaz non condensés sont lavés dans un scrubber à eau avant de balayer une ampoule de prélèvement. Le bilan des produits formés est effectué au départ d'analyses chromatographiques des produits liquide et gazeux recueillis et du titrage de l'acidité de la solution aqueuse recueillie au pied du scrubber.

6 essais sont réalisés avec des compositions catalytiques contenant des quantités différentes de lithium telles que reprises au Tableau 2 ci-après. Ce Tableau 2 comprend également les conditions de fonctionnement du réacteur et les résultats obtenus.

Les rendements obtenus dans ces essais de laboratoire sont limités par le temps de séjour restreint de 5 s. La conversion de $C_2H_4$ est, dès lors, limitée dans tous les essais (les conditions de marche d'un réacteur industriel permettent d'atteindre des temps de séjour nettement plus longs : 10 à 80 s, et le plus souvent de 20 à 50 s afin d'assurer une meilleure conversion de l'éthylène).

6

Tableau 2

| ESSAI | 5 | 6 | 7 | 8 | 9 | 10 |
|---|---|---|---|---|---|---|
| I. Catalyseur | | | | | | |
| a) support : | | | | | | |
| alumine : type | γ | γ | γ | γ | γ | γ |
| : surf. spécifique (m²/g) | 190 | 190 | 180 | 180 | 180 | 180 |
| b) composition catalytique | | | | | . | |
| Cu en g/kg | 59 | 60 | 59,6 | 58,1 | 56,5 | 56,9 |
| Mg en g/kg | 16,6 | 16,1 | 16,1 | 16,2 | 13,6 | 14,5 |
| Li en g/kg | 0,3 | 1 | 2,1 | 4,2 | 11,2 | 21,4 |
| Surface spécifique BET m²/g | 117 | 123 | 99 | 94 | 88 | 67 |
| II. Conditions opératoires de l'oxychloration | | | | | | |
| HCl : 1N/h | 160 | 160 | 160 | 160 | 160 | 160 |
| air : 1N/h | 260 | 260 | 260 | 260 | 260 | 260 |
| $C_2H_4$ : 1N/h | 84 | 84 | 84 | 84 | 84 | 84 |
| t° : °C * | 255 | 255 | 255 | 255 | 255 | 255 |
| pression : bars absolus | 6 | 6 | 6 | 6 | 6 | 6 |
| vitesse de fluidisation (cm/s) | 10 | 10 | 10 | 10 | 10 | 10 |
| temps de contact : s | 5 | 5 | 5 | 5 | 5 | 5 |

* Profil de température stable dans le réacteur : la température du lit fluide ne s'écarte pas de plus de 2°C de la température moyenne.

7

Tableau 2 (suite)

| ESSAI | 5 | 6 | 7 | 8 | 9 | 10 |
|---|---|---|---|---|---|---|
| RENDEMENT : | | | | | | |
| 1,2-DCEa (% mol) | | | | | | |
| $C_2H_4$ mis en oeuvre | 91,5 | 91,6 | 91,7 | 88 | 83 | nd |
| Conversion de $C_2H_4$ % mol. | 97 | 96,4 | 95,4 | 91,2 | 88 | nd |
| Formation de : $C_2H_5Cl$ % mol. par rapport au $C_2H_4$ mis en oeuvre | 0,7 | 0,6 | 1,15 | 2,25 | 3,25 | - |
| Corrosion de l'acier AISI 316 L | faible | faible | faible | faible | - | - |
| | fig.5 | fig.6 | fig.7 | fig.8 et 9 | fig. 10 et 11 | fig. 12 et 13 |

nd : non déterminé.

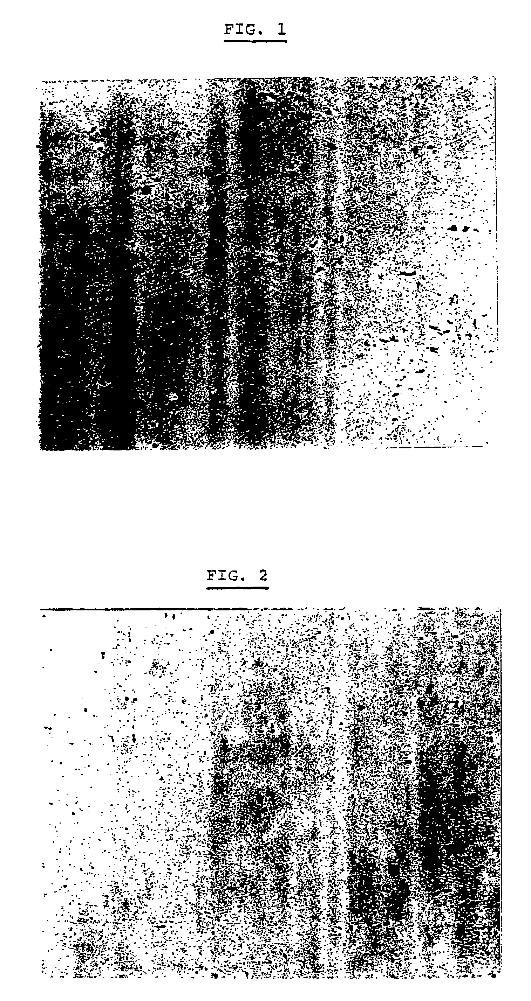

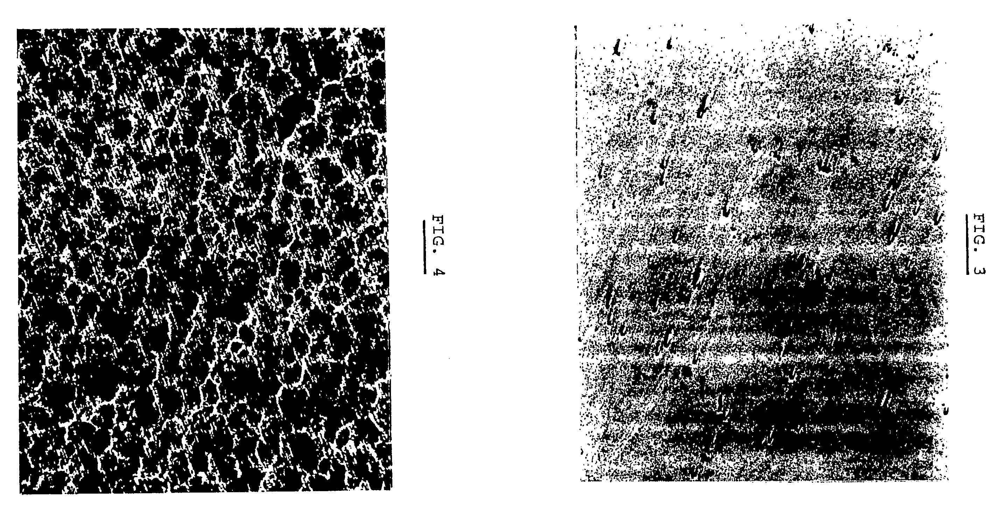

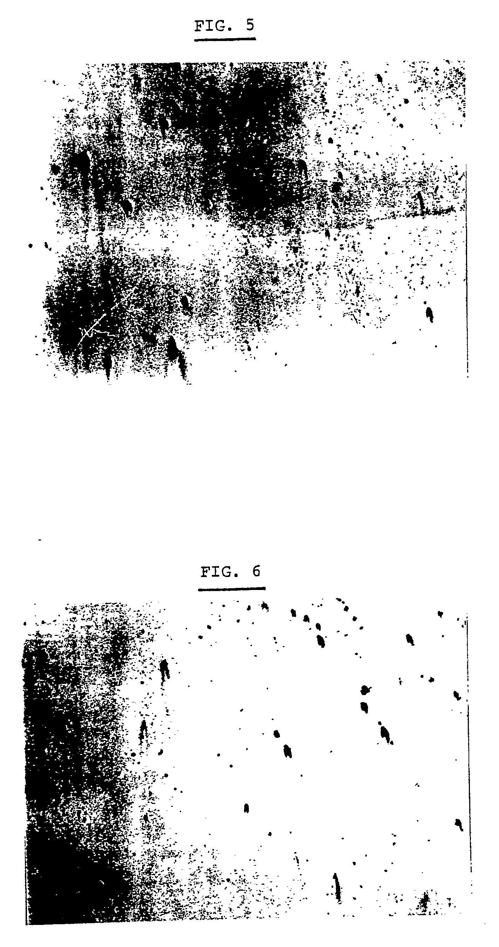

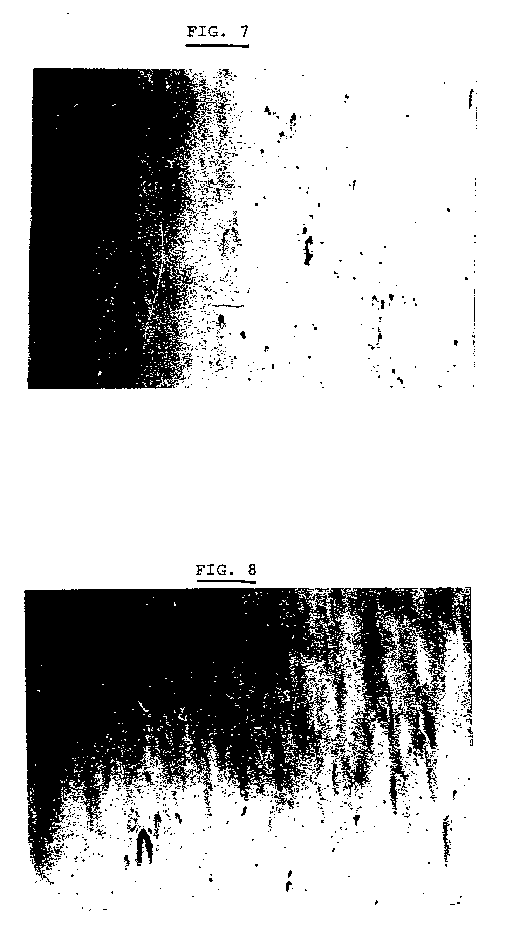

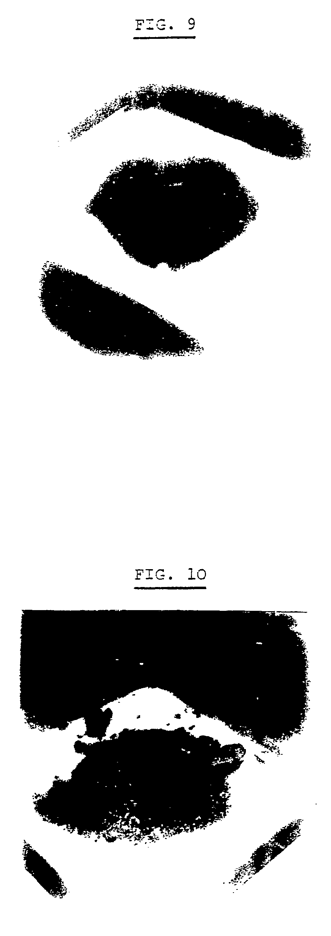

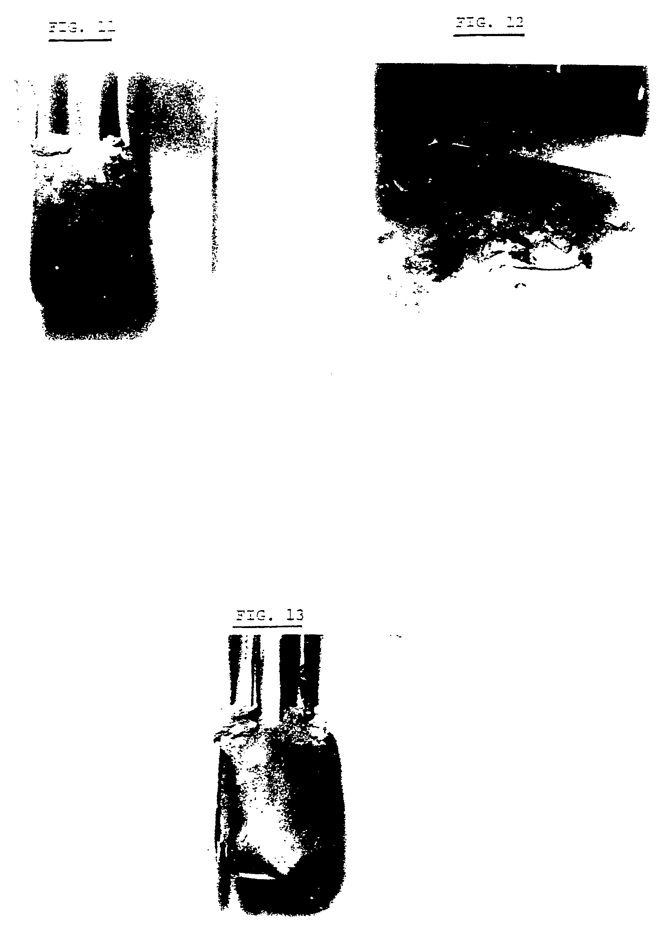

On peut déduire de la comparaison des résultats des différents essais que lorsque l'on opère au dessus d'une certaine limite en lithium, le rendement en 1,2-dichloréthane chute vite (essais 8 et 9) bien que la corrosion de l'acier soit faible mais devient inacceptable du fait du collage et mottage des grains de catalyseur (figure 9). On peut également déduire des résultats des différents essais que lorsque l'on dépasse les limites en lithium des catalyseurs selon l'invention, les catalyseurs deviennent inutilisables en pratique puisque dans ce cas on obtient une forte tendance au collage et au mottage (fig. 10 et fig. 12), ce qui rend les mesures de corrosion dans un érosimètre impossibles (fig. 11 et fig. 13).

**Revendications**

1. Procédé d'oxychloration d'éthylène en 1,2-dichloréthane catalysé par une composition catalytique comprenant un support d'alumine sur lequel est déposé du chlorure cuivrique, du chlorure de magnésium et un chlorure d'un métal alcalin, caractérisé en ce que :
   a) le chlorure de métal alcalin est choisi parmi le chlorure de sodium et le chlorure de lithium,
   b) lorsque le métal alcalin de la composition catalytique est le sodium, il est mis en oeuvre dans un rapport atomique par rapport au cuivre compris entre 0,01-0,2 : 1 et
   c) lorsque le métal alcalin de la composition catalytique est le lithium, il est mis en oeuvre dans un rapport atomique par rapport au cuivre compris entre 0,0001-2 : 1.

2. Procédé selon la revendication 1 caractérisé en ce que l'on met en oeuvre une composition catalytique comprenant un support d'alumine sur lequel sont déposés du chlorure cuivrique, du chlorure de magnésium et du chlorure de sodium dans des rapports atomiques :
   Na : Mg : Cu = 0,01-0,2 : 0,1-1,5 : 1

3. Procédé selon la revendication 2 caractérisé en ce que ces rapports atomiques sont :

Na : Mg : Cu = 0,05-0,08 : 0,74-0,80 : 1

4. Procédé selon la revendication 3 caractérisé en ce que les rapports atomiques sont Na : Mg : Cu = 0,05 : 0,74 : 1

5. Procédé selon la revendication 3 caractérisé en ce que les rapports atomiques sont Na : Mg : Cu = 0,08 : 0,80 : 1

6. Procédé selon la revendication 1 caractérisé en ce que l'on met en oeuvre une composition catalytique comprenant un support d'alumine sur lequel sont déposés du chlorure cuivrique, du chlorure de magnésium et du chlorure de lithium dans des rapports atomiques :
Li : Mg : Cu = 0,0001-1,5 : 0,1-1,5 : 1

7. Procédé selon la revendication 6 caractérisé en ce que les rapports atomiques sont Li : Mg : Cu = 0,001-1 : 0,3-1 : 1

8. Compositions catalytiques pour l'oxychloration d'hydrocarbures comprenant du chlorure cuivrique, du chlorure de magnésium et du chlorure de sodium dans des rapports atomiques compris entre 1 : 0,1-1,5 : 0,01-0,2 déposés sur alumine.

9. Compositions catalytiques pour l'oxychloration d'hydrocarbures comprenant du chlorure cuivrique, du chlorure de magnésium et du chlorure de lithium dans de rapports atomiques compris entre 1 : 0,1-1,5 : 0,0001-1,5 déposés sur alumine.

## Claims

1. Process for the oxychlorination of ethylene to 1,2-dichloroethane, catalysed by a catalyst composition comprising an alumina support on which cupric chloride, magnesium chloride and an alkali metal chloride are deposited, characterised in that:
a) the alkali metal chloride is chosen from sodium chloride and lithium chloride,
b) when the alkali metal of the catalyst composition is sodium, it is used in an atomic ratio in relation to copper of between 0.01 - 0.2 : 1 and
c) when the alkali metal of the catalyst composition is lithium, it is used in an atomic ratio in relation to copper of between 0.0001 - 2 : 1.

2. Process according to Claim 1, characterised in that a catalyst composition is used comprising an alumina support on which cupric chloride, magnesium chloride and sodium chloride are deposited in atomic ratios:
Na : Mg : Cu = 0.01 - 0.2 : 0.1 - 1.5 : 1.

3. Process according to Claim 2, characterised in that these atomic ratios are:
Na : Mg : Cu = 0.05 - 0.08 : 0.74 - 0.80 : 1.

4. Process according to Claim 3, characterised in that the atomic ratios are Na : Mg : Cu = 0.05 : 0.74 : 1.

5. Process according to Claim 3, characterised in that the atomic ratios are Na : Mg : Cu = 0.08 : 0.80 : 1.

6. Process according to Claim 1, characterised in that a catalyst composition is used comprising an alumina support on which cupric chloride, magnesium chloride and lithium chloride are deposited in atomic ratios:
Li : Mg : Cu = 0.0001 - 1.5 : 0.1 - 1.5 : 1.

7. Process according to Claim 6, characterised in that the atomic ratios are Li : Mg : Cu = 0.001 - 1 : 0.3 - 1 : 1.

8. Catalyst compositions for the oxychlorination of hydrocarbons, comprising cupric chloride, magnesium chloride and sodium chloride in atomic ratios of between 1 : 0.1 - 1.5 : 0.01 - 0.2 deposited on alumina.

9. Catalyst compositions for the oxychlorination of hydrocarbons, comprising cupric chloride, magnesium chloride and lithium chloride in atomic ratios of between 1 : 0.1 - 1.5 : 0.0001 - 1.5 deposited on alumina.

**Patentansprüche**

1. Verfahren der Oxychlorierung von Ethylen zu 1,2-Dichlorethan katalysiert durch eine katalytische Zusammensetzung, die einen Aluminiumoxidträger umfaßt, an dem Kupferchlorid, Magnesiumchlorid und ein Alkalimetallchlorid angelagert ist, dadurch gekennzeichnet, daß
   a) das Alkalimetallchlorid ausgewählt ist aus Natriumchlorid und Lithiumchlorid,
   b) wenn das Alkalimetall der katalytischen Zusammensetzung Natrium ist, es in einem atomaren Verhältnis bezüglich des Kupfers zwischen 0,01-0,2 : 1 eingesetzt wird und
   c) wenn das Alkalimetall der katalytischen Zusammensetzung Lithium ist, es in einem atomaren Verhältnis bezüglich des Kupfers zwischen 0,0001-2 : 1 eingesetzt wird.

2. Verfahren nach Anspruch 1, dadurch gekennzeichnet, daß man eine katalytische Zusammensetzung einsetzt, die einen Aluminiumoxidträger umfaßt, auf dem Kupferchlorid, Magnesiumchlorid und Natriumchlorid in atomaren Verhältnissen von:
   Na : Mg : Cu = 0,01-0,2 : 0,1-1,5 : 1 angelagert sind.

3. Verfahren nach Anspruch 2, dadurch gekennzeichnet, daß die atomaren Verhältnisse
   Na : Mg : Cu = 0,05-0,08 : 0,74-0,80 : 1 sind.

4. Verfahren nach Anspruch 3, dadurch gekennzeichnet, daß die atomaren Verhältnisse
   Na : Mg : Cu = 0,05 : 0,74 : 1 sind.

5. Verfahren nach Anspruch 3, dadurch gekennzeichnet, daß die atomaren Verhältnisse
   Na : Mg : Cu = 0,08 : 0,80 : 1 sind.

6. Verfahren nach Anspruch 1, dadurch gekennzeichnet, daß man eine katalytische Zusammensetzung einsetzt, die einen Aluminiumoxidträger umfaßt, an dem Kupferchlorid, Magnesiumchlorid und Lithiumchlorid in den atomaren Verhältnissen:
   Li : Mg : Cu = 0,0001-1,5 : 0,1-1,5 : 1 angelagert sind.

7. Verfahren nach Anspruch 6, dadurch gekennzeichnet, daß die atomaren Verhältnisse
   Li : Mg : Cu = 0,001-1 : 0,3-1 : 1 sind.

8. Katalytische Zusammensetzungen zur Oxychlorierung von Kohlenwasserstoffen, die Kupferchlorid, Magnesiumchlorid und Natriumchlorid in atomaren Verhältnissen zwischen 1 : 0,1-1,5 : 0,01-0,2 angelagert an Aluminiumoxid umfassen.

9. Katalytische Zusammensetzungen zur Oxychlorierung von Kohlenwasserstoffen, die Kupferchlorid, Magnesiumchlorid und Lithiumchlorid in atomaren Verhältnissen zwischen 1 : 0,1-1,5 : 0,0001-1,5 angelagert an Aluminiumoxid umfassen.

FIG. 1

FIG. 2

FIG. 3

FIG. 4

FIG. 5

FIG. 6

## FIG. 7

## FIG. 8

FIG. 9

FIG. 10

FIG. 11

FIG. 12

FIG. 13